# EUROPEAN PATENT SPECIFICATION

(11) **EP 1 144 007 B1**
(45) Date of publication and mention of the grant of the patent: **20.09.2006**
(21) Application number: 00901377.2
(22) Date of filing: 13.01.2000
(51) Int. Cl.: A61K 39/395, C07B 61/00, C07B 63/00, G01N 33/531

(54) **MOLECULARLY IMPRINTED MICROSPHERES PREPARED USING PRECIPITATION POLYMERISATION**
DURCH PRÄZIPITATIONSPOLYMERISATION HERGESTELLTE MOLEKULAR MARKIERTE MIKROKUGELN
MICROSPHERES A EMPREINTES MOLECULAIRES PREPARES PAR POLYMERISATION DE DESURSATURATION

(30) Priority: 14.01.1999 SE 9900121
(43) Date of publication of application: 17.10.2001
(73) Proprietor: MOSBACH, Klaus, S-244 94 Furulund (SE)
(72) Inventor: MOSBACH, Klaus, S-244 94 Furulund (SE); YE, Lei, S-226 46 Lund (SE); CORMACK, Peter A. G., Glasgow G42 9TG (GB)
(74) Representative: Stenbäck, Maria Elisabeth
(86) International application number: PCT/SE2000/000047
(87) International publication number: WO 2000/041723

(56) References cited:
- WO-A1-97/38015
- KAI LI ET AL.: 'Synthesis of Monodisperse Poly(divinylbenzene) Microspheres' JOURNAL OF POLYMER SCIENCE: PART A: POLYMER CHEMISTRY, vol. 31, 1993, pages 3257 - 3263, XP002928405

## Description

The present invention relates to molecularly imprinted microsperes, to a method of producing said microspheres and to the use of said microspheres.

More particularly, the present invention relates to a method for preparing molecularly imprinted microspheres in the absence of any added surfactants. Highly specific, molecularly imprinted microspheres in the micron-scale size range can be produced quickly, cleanly and in excellent yield by this method, and the regular particle size and shape of the microspheres obtained is advantageous in several ways. These artificial receptors can readily replace biologically derived receptors in many applications and are therefore highly attractive. Several possible applications are described herein.

### BACKGROUND OF THE INVENTION

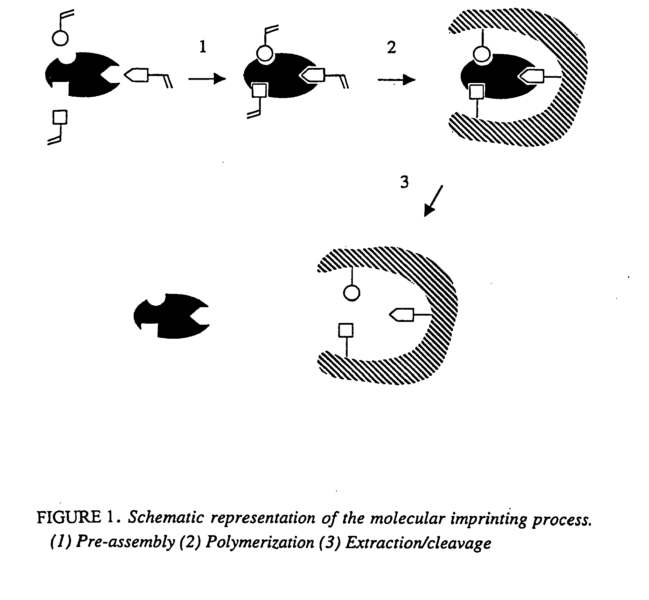

Molecular imprinting is an established technique for the preparation of synthetic receptors with high affinities and specificities for various analytes of interest. During the free-radical polymerisation commonly used in the imprinting process, the incorporation of template-complementary functionality into the polymer matrix, which is the key to ligand re-binding, is guided by the template molecules themselves, since they form complementary guest-host complexes with the functional monomers. Following removal of the template from the polymer matrix, the crosslinked polymeric host can rebind the original template very specifically (Figure 1) [1-3].

Depending on the nature of the interactions guiding the assembly of the guest-host complex and the subsequent recognition of the target ligand, molecular imprinting strategies can be divided into two major categories: covalent and non-covalent imprinting approaches. A semi-covalent imprinting method is also reported, where one can use covalent interactions for the preparation of the imprinted polymer and non-covalent interactions for the subsequent re-binding of ligands of interest [4].

These molecularly imprinted receptor analogs are easy to produce and very stable, and are therefore superior to natural receptors in many respects.

Molecularly imprinted polymers have been used for chromatographic separation [5], in biomimetic sensors [6], in catalyzing chemical reactions [7], in solid phase extraction for sample enrichment/clean-up [8], in screening of combinatorial chemical libraries [9], for in situ product removal during biotransformation processes [10], and down-stream product purification [11]. They also have great potential for drug determination using for instance ligand competition assays [12].

Imprinted polymers are usually prepared in the form of a monolith which is then ground and sieved to the desired particle size. The grinding and sieving process is time-consuming and yields only moderate amounts of useful imprinted polymer. The polymer particles obtained are also irregularly-shaped, which is not ideal for chromatographic purposes. Furthermore, the grinding process may also be detrimental to some of the binding sites.

Suspension polymerisation in perfluorocarbon liquid continuous phases has been introduced to address some of these issues [13]. Although this method delivers good yields of spherical particles with controlled particle sizes, it is not a straightforward method in that it requires considerable optimisation. The perfluorocarbon dispersing phase is also somewhat expensive.

Other imprinting methods leading to spherical particles with controlled sizes include emulsion polymerisation in aqueous media, and seeded emulsion polymerisation. However, they involve either the use of stabilisers or multi-step operations, which are neither straightforward nor broadly applicable for imprinting.

### BRIEF SUMMARY OF THE INVENTION

The present invention provides a new method of producing molecularly imprinted microspheres. Typically, the diameters of the microspheres are between 0.01 - 10 µm. The method is based on surfactant-free precipitation polymerisation. The specific binding sites are created using print molecules that form either non-covalent, reversible covalent or "semi-covalent" interactions with the functional monomers as templates.

In the method the total volume of the polymerisable monomer/crosslinker is typically kept within 0.01 - 20% v/v that of the reaction solvent. The reaction solvent employed is either aqueous or non-aqueous, and is either single component or composed of multiple solvents.

The interactions between print molecules and functional monomers utilised for imprinting and rebinding can be either reversible covalent, non-covalent, or both. Multiple interactions of different characters can be simultaneously utilised. Different functional monomers can be employed simultaneously, in addition to using single functional monomers.

The solubility of the print molecules in the reaction solvent can be adjusted by changing the composition of the reaction solvent.

The polymerisation can be induced by heat, by UV, by γ radiation or by chemical methods. Free-radical polymerisation, ionic polymerisation, coordination polymerisation, step growth polymerisation or related methods are used to prepare molecularly imprinted microspheres without using surfactant.

Microspheres with desired particle sizes can be produced by controlling the nucleation and particle growth process of the resulting polymer. This is achieved through adjusting the composition of functional monomer/crosslinker/solvent system, as well as reaction conditions, in order to change the solubility of the growing polymer chains. The polymerisation conditions are controlled in such a way as to avoid aggregation of the microspheres.

The molecularly imprinted microspheres can be used as replacements for conventionally imprinted polymers in various applications. Thus, they can be used for the screening of chemical libraries, for catalysis, for facilitated synthesis, for analyte determination using competitive ligand binding assays and agglutination assays.

The microspheres can also be used as stationary phase or modifier in capillary electrophoresis, capillary electrochromatography and HPLC analysis, as recognition component in biomimetic sensors, as affinity-labelled probe for targeting cells or other biological materials.

A further use of the molecularly imprinted microspheres is as binding entities for the preparation of composite materials.

### BRIEF DESCRIPTION OF THE DRAWINGS

Figure 1 is a reaction scheme of a prior art molecular imprinting process.
Figure 2 shows some examples of functional monomers which can be used in the process according to the invention.
Figure 3 shows electron micrographs of anti-17β-estradiol microspheres prepared according to Example 3.
Figure 4 shows the displacement of radioligand binding to molecularly imprinted microspheres under equilibirium conditions, as disclosed in Example 4. B/B₀ is the ratio of the amount of radioligand bound in the presence of displacing ligand, B, to the amount bound in the absence of displacing ligand, B₀.
Figure 5 shows a calibration curve for theophylline, as disclosed in Example 5.
Figure 6 shows the specificity of the anti-17β-estradiol microspheres prepared according to Example 3.

### DETAILED DESCRIPTION OF THE INVENTION

The present invention relates to a novel method for preparing molecularly imprinted microspheres using precipitation polymerisation, the microspheres as obtained by said method, and the applications of these imprinted microspheres.

Precipitation polymerisation, sometimes called surfactant-free polymerisation, can be used to prepare mono-disperse microspheres with controlled particle diameters typically within the 0.1-10 µm range [14-17]. The mechanism for particle formation and growth resembles that of dispersion polymerisation, except that the particles are stabilised against coagulation by their rigid, crosslinked surfaces, rather than by any added stabilisers. These microspheres are easy to prepare and are free from any adsorbed surfactants. Significantly, neither polymer grinding nor sieving steps are necessary following polymerisation, therefore the preparation of molecularly imprinted microspheres by this method is much more efficient in terms of yield and much less time-consuming to perform.

In conventional molecular imprinting protocols, the yield of imprinted polymer with the desired particle size range following successive grinding and sieving operations is usually less than 50%. In contrast, the present method allows polymer yields upwards of 85% to be attained. The regular size and shape of the particles can facilitate system homogenisation and is advantageous for mass transfer in ligand rebinding processes. It also offers benefits in chromatographic applications.

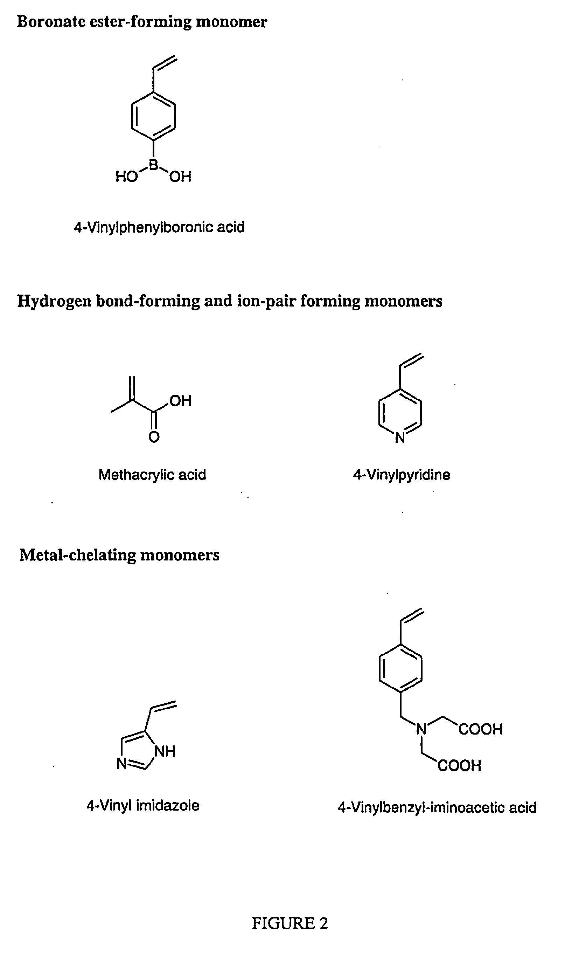

Both reversible covalent and non-covalent interactions can be utilised during the imprinting process when using precipitation polymerisation. The semi-covalent strategy can also be used. Functional monomers for reversible covalent interactions include boronate ester-forming monomers, Schiff base-forming monomers, and carbonate-forming monomers. For non-covalent interactions, hydrogen bond-forming monomers, ion-pair forming monomers, metal-chelating monomers, as well as hydrophobic monomers can be used (Figure 2).

Various crosslinkers can be used depending on the solvent employed as a porogen.

Polymerisation can be initiated in a variety of ways, typically via thermal or photochemical means, and both water-soluble and organic solvent miscible initiators can be used, depending on the solvents employed.

To obtain spherical microspheres with good recognition behaviour, efficient crosslinking has to be ensured. Typically this is achieved by using a high degree of crosslinking. For some purposes, however, a much lower crosslinking density can still yield microspheres with satisfactory molecular recognition capabilities.

Compared to conventional imprinting methods, far greater amounts of solvent are used in precipitation polymerisation protocols to prepare the imprinted microspheres. Both aqueous and non-aqueous solvents can be used for different target print molecules. When the non-covalent strategy is used, except where the hydrophobic effect is of interest, less-polar organic solvents, for example dichloromethane and acetonitrile are generally most satisfactory. Imprinting via other strategies can readily use aqueous and polar non-aqueous solvents. The total monomer volume in the polymerization solution is generally within the range of 1 - 10% v/v with respect to the polymerisation solvent, to prevent aggregation of the microspheres.

The amount of print molecule can be, though not necessarily, so high as to saturate the solvent containing the functional monomer and the crosslinker at the polymerisation temperature in order to provide a high load capacity for the resulting imprinted microspheres.

On the other hand, a poor solvent for the print molecule can be introduced as a co-solvent, if required, to reduce the solubility of the print molecule and therefore to reduce the amount of print molecule required. This may be an attractive approach when one is using an expensive print molecule, for example, hexane can be added to acetonitrile to make the print molecule much less soluble while the complex formation between the functional monomer and the print molecule in the non-covalent approach is not sacrificed.

By controlling various reaction conditions, such as the solubility parameters of the resulting polymer and that of the solvent, the nucleation and growth behaviour of the polymer particles can be tailored to deliver microspheres of controlled particle diameter and porosity that retain high affinity and specificity for the print molecules.

The molecularly imprinted microspheres can be used in various applications. These artificial receptors can readily replace their natural counterparts in many instances. Their regular size and shape allows better reproducibility in different assays. Non-limiting examples of applications of molecularly imprinted microspheres, including monodisperse microspheres, are: 1) as stationary phases or modifiers in capillary electrophoresis; 2) as recognition components in biomimetic sensors; 3) as catalysts to facilitate chemical/biochemical reactions; 4) as probes for cell or other biological material targeting in which case they are dyed or made magnetic; 5) for drug determination using competitive ligand assay; 6) as bio-compatible carrier for controlled drug release; 7) as binding components to prepare composite materials for affinity purification/isolation of target compounds.

The invention will now be described more in detail by way of the following non-limiting examples.

### EXAMPLE 1

### Preparation of anti-theophylline microspheres

Acetonitrile (50 mL) is mixed with methacrylic acid (MAA, 372.5 mg) and trimethylolpropane trimethacrylate (TRIM, 627.5 mg) in a borosilicate glass tube. Theophylline (115 mg) is suspended in the solution and dissolved after sonication at 60°C. The initiator, azobisisobutyronitrile (AIBN, 17.5 mg) is dissolved, the solution purged with nitrogen for five minutes and the tube sealed under nitrogen. Polymerisation is induced by placing the tube in a water bath preset at 60°C and continued for 24 hours.

The microspheres formed are collected by centrifugation at 8000 rpm for 10 minutes using a RC5C superspeed refrigerated centrifuge from BECKMAN (Palo Alto, CA, USA). The print molecule is thoroughly extracted by washing repeatedly with methanol containing 10% acetic acid (v/v), followed by a final wash in acetone. These successive centrifugation and decanting steps extract the print molecule from the polymer. The anti-theophylline microspheres obtained are monodisperse and have an average diameter of 0.2 µm. The microspheres are finally dried *in vacuo.* The reference (control) microspheres are prepared and treated in exactly the same way, except that no print molecule is used in the polymerisation stage.

### EXAMPLE 2

### Preparation of anti-theophylline microspheres

Acetonitrile (50 mL) is mixed with MAA (372.5 mg) and TRIM (627.5 mg) in a borosilicate glass tube. Theophylline (11.5 mg) and AIBN (17.5 mg) are dissolved in the solution. The solution is purged with nitrogen for five minutes and the tube sealed under nitrogen. Polymerisation is induced by UV irradiation (350 nm) at 20°C using a RMA-400 Rayonet photochemical reactor from Southern New England Ultraviolet Co. (Bradford, CT, USA) and continued for 24 hours.

The microspheres obtained are treated in the same way as in example 1 to remove the print molecule. The reference (control) microspheres are prepared and treated in exactly the same way, except that no print molecule is used in the polymerisation stage.

### EXAMPLE 3

### Preparation of anti-17β-estradiol microspheres

Acetonitrile (50 mL) is mixed with MAA (372.5 mg) and TRIM (627.5 mg) in a borosilicate glass tube. 17β-Estradiol (250 mg) and AIBN (17.5 mg) are dissolved in the above solution. The solution is purged with nitrogen for five minutes and the tube sealed under nitrogen. Polymerisation is induced by UV irradiation (350 nm) at 20°C using a RMA-400 Rayonet photochemical reactor from Southern New England Ultraviolet Co. (Bradford, CT, USA) and continued for 24 hours.

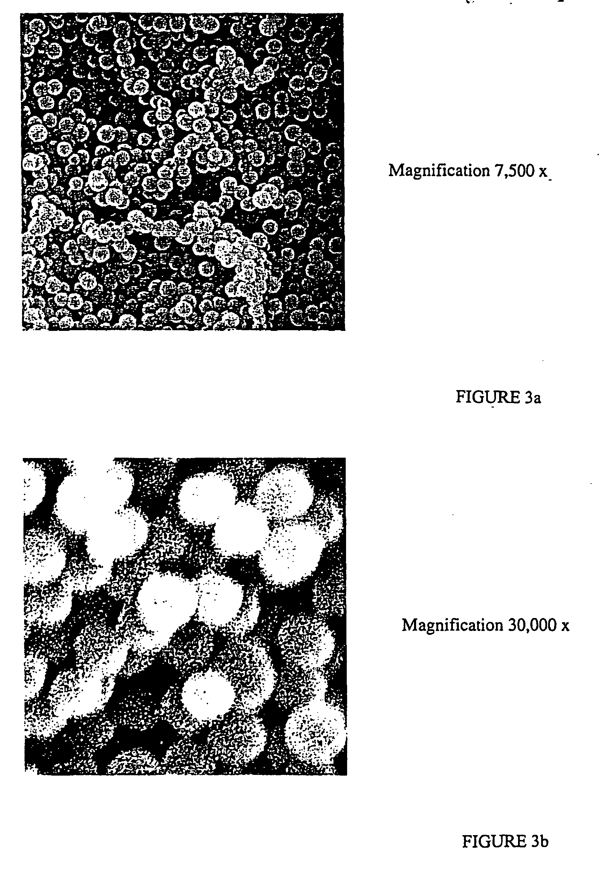

The microspheres obtained are treated in the same way as per example 1 to remove the print molecule. The anti-17β-estradiol microspheres obtained are monodisperse and have an average diameter of 0.3 µm (Figure 3). The reference (control) microspheres are prepared and treated in exactly the same way, except that no print molecule is used in the polymerization stage.

### EXAMPLE 4

### Competitive radioligand assay using anti-theophylline microspheres from example 1

The binding capacity of the anti-theophylline microspheres from example 1 is estimated from saturation studies. Varying amounts of the microspheres are incubated overnight and at room temperature with 16.2 pmol (685 Bq) [8-³H]theophylline in 1 mL acetonitrile, using polypropylene microcentrifuge tubes. A rocking table ensured gentle mixing.

The microspheres are then separated by centrifugation at 14,000 rpm for five minutes, 500 µL supernatant mixed with 10 mL scintillation liquid, Ecoscint O (National Diagnostics, Manville, NJ, USA), and the radioactivity then measured using a model 2119 RACKBETA β-radiation counter from LKB Wallac (Sollentuna, Sweden). The amount of anti-theophylline microspheres required to bind half of the added radioligand is estimated to be 5 mg, while an equivalent amount of the reference polymer binds less than 10% of the added radioligand.

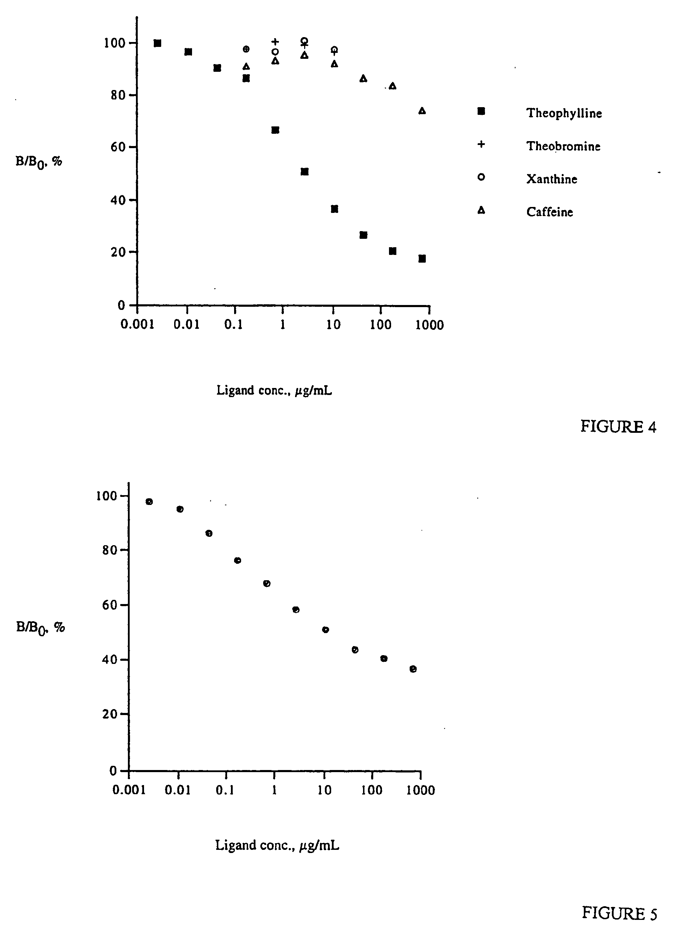

The theophylline-imprinted microspheres are suspended in acetonitrile (25 mg/mL) and sonicated to form a polymer stock suspension, from which 200 µL was transferred into each microcentrifuge tube. Varying amounts of non-radiolabelled ligand, including theophylline, theobromine, xanthine and caffeine, and 16.2 pmol (685 Bq) [8-³H]theophylline are added, and the final volume adjusted to 1 mL with acetonitrile. The competitive binding is allowed to proceed overnight by incubation at ambient temperature, using a rocking table for gentle mixing. The amount of bound radioligand is estimated by measuring the radioactivity from 500 µL supernatant following centrifugation at 14,000 rpm for five minutes. The high specificity of the anti-theophylline microspheres can be evaluated by comparing the IC₅₀ value of compounds closely related to the print molecule, with IC₅₀ being the ligand concentration that can displace 50% of the bound radioligand from the imprinted microspheres. Figure 4 shows the displacement of radioligand binding to molecularly imprinted microspheres under equilibrium condition. B/B₀ is the ratio of the amount of radioligand bound in the presence of displacing ligand, B, to the amount bound in the absence of displacing ligand, B₀.

### EXAMPLE 5

### Competitive radioligand assay using anti-theophylline microspheres from example 2

The same procedure as used in example 4 is followed, except that the microspheres are from example 2. The amount of anti-theophylline microspheres required to bind half of the added radioligand is estimated to be 10 mg, while an equivalent amount of the reference polymer binds less than 20% of the added radioligand.

The theophylline-imprinted microspheres are suspended in acetonitrile (50 mg/mL) and sonicated to form a polymer stock suspension, from which 200 µL was transferred into each microcentrifuge tube. The same competitive binding assay as in example 4, using theophylline as the cold ligand, is followed. A calibration curve for theophylline similar to the one in Figure 4 is obtained, although the non-specific binding is slightly higher (Figure 5).

### EXAMPLE 6

### Competitive radioligand assay using anti-17β-estradiol microspheres from example 3

Varying amounts of the microspheres were incubated overnight and at room temperature with 417 fmol (1110 Bq) [2,4,6,7-³H(N)] estradiol in 1 mL acetonitrile, using polypropylene microcentrifuge tubes. Other conditions are the same as used in example 4. The amount of anti-17β-estradiol microspheres required to bind half of the added radioligand is estimated to be 30 mg, while an equivalent amount of the reference polymer binds less than 12% of the added radioligand.

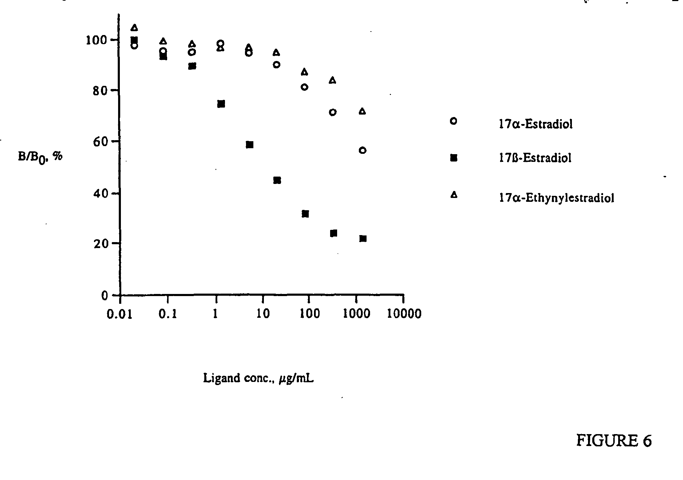

The 17β-estradiol-imprinted microspheres are suspended in acetonitrile (150 mg/mL) and sonicated to form a polymer stock suspension, from which 200 µL was transferred into each microcentrifuge tube. Varying amounts of non-radiolabelled ligand, including 17β-estradiol, 17α-estradiol and 17α-ethynylestradiol, and 417 fmol (1110 Bq) [2,4,6,7-³H(N)] estradiol are added, and the final volume adjusted to 1 mL with acetonitrile. Other conditions are the same as used in example 4. The specificity of the anti-17β-estradiol microspheres is signified in Figure 6.

### REFERENCES

(1) Mosbach, K.; Ramström, O. *Bio*/*Technology* 1996, *14*, 163-170.
(2) Wulff, G. *Angew. Chem. Int. Ed. Engl.* 1995, *34,* 1812-1832.
(3) Shea, K. J. *Trends in Polymer Science* 1994, *2,* 166-173.
(4) Whitcombe, M.; Rodriguez, M.; Villar, P.; Vulfson, E. *J. Am. Chem. Soc.* 1995, *117,* 7105-7111.
(5) Kempe, M. *Anal. Chem.* 1996, *68*, 1948-1953.
(6) Kriz, D.; Ramström, O; Svensson, A; Mosbach, K. *Anal. Chem.* 1995, *67*, 2142-2144.
(7) Müller, R.; Andersson, L. I.; Mosbach, K. *Makromol. Chem. Rapid Commun.* 1993, 14, 637-641.
(8) Sellergren, B. *Anal. Chem.* 1994, *66*, 1578-1582.
(9) Ramström, O.; Ye, L.; Krook, M.; Mosbach, K. *Anal. Commun.* 1998, *35*, 9-11.
(10) Ye, L.; Ramström, O.; Månsson, M.-O.; Mosbach, K. *J. Mol. Reg.* 1998, *11*, 1-4.
(11) Ye, L.; Ramström, O.; Mosbach, K. *Anal. Chem.* 1998, 70, 2789-2795.
(12) Vlatakis, G.; Andersson, L. I.; Müller, R.; Mosbach, K. *Nature* 1993, *361*, 645-647.
(13) Mayes, A.; Mosbach, K. *Anal. Chem.* 1996, *68,* 3769-3774.
(14) Naka, Y.; Kaetsu, I.; Yamamoto, Y.; Hayashi, K. *J. Polym. Sci.: Part A: Polym. Chem.* 1991, *29,* 1197-1202.
(15) Naka, Y.; Yamamoto, Y. *J. Polym. Sci.: Part A: Polym. Chem.* 1992, *30*, 1287-1298.
(16) Li, K.; Stöver, D. H. *J. Polym. Sci.: Part A: Polym. Chem.* 1993, *31*, 3257-3263.
(17) Li, W.-H.; Stöver, D. H. *J. Polym. Sci.: Part A: Polym. Chem.* 1998, *36*, 1543-1551.

## Claims

1. A method of producing molecularly imprinted microspheres comprising specific binding sites,
**characterised by** polymerising functional monomers and crosslinkers in a reaction solvent in the presence of print molecules as templates in a surfactant-free precipitation polymerisation process, which print molecules are capable of forming non-covalent or reversible covalent interactions with said functional monomers.

2. A method according to claim 1, wherein the total volume of polymerisable monomers and crosslinkers is kept in the range of about 0.01 to 20 % of the volume of the reaction solvent.

3. A method according to claim 1 or 2, wherein the reaction solvent is aqueous or non-aqueous.

4. A method according to claim 1 or 2, wherein said reaction solvent is composed of a single solvent component or of multiple solvent components.

5. A method according to claim 1, wherein said functional monomers have the same functionality.

6. A method according to claim 1, wherein said functional monomers have different functionality.

7. A method according to claim 1 or 2, wherein the solubility of the print molecules in the reaction solvent is adjusted by changing the composition of the reaction solvent.

8. A method according to claim 1, wherein the polymerisation is induced by heat, UV radiation, γ radiation and/or chemically.

9. A method according to claim 1, wherein said polymerisation process is a free-radical polymerisation process, an ionic polymerisation process, a coordination polymerisation process or a step growth polymerisation process.

10. A method according to claim 1 or 2, wherein a desired size of the microspheres is achieved by controlling the nucleation and particle growth process.

11. A method according to claim 10, wherein the control of the nucleation and particle growth process is achieved by adjusting the composition of the functional monomer/crosslinker/solvent system and/or the reaction conditions during the polymerisation in order to change the solubility of the growing polymer chains.

12. A method according to claim 10, wherein the control of the nucleation and particle growth process is such as to avoid aggregation of the microspheres.

13. A method according to claim 1 or 2, wherein the size of the microspheres as produced is in the range of 0.01-10µm.

14. A method according to claim 1 or 2, wherein the reaction conditions are controlled so that the microsperes become monodisperse.

15. In vitro use of the molecularly imprinted microspheres as prepared according to any one of claims 1-14, for screening of chemical libraries, for catalysis, for facilitating synthesis, for analyte determination using ligand binding assays and/or agglutination assays, for therapeutic purposes, or for controlled release.

16. Use of the molecularly imprinted microspheres as prepared according to any one of claims 1-14, as stationary phase or modifier in capillary electrophoresis, capillary electrochromatography or HPLC analysis.

17. Use of the molecularly imprinted microspheres as prepared according to any one of claims 1-14, as recognition component in biomimetic sensors.

18. In vitro use of the molecularly imprinted microspheres as prepared according to any one of claims 1-14, as affinity-labelled probe for targeting cells or other biological material.

19. Use of the molecularly imprinted microspheres as prepared according to any one of claims 1-14, as binding entities for the preparation of composite materials.

## Patentansprüche

1. Verfahren zum Herstellen von molekular geprägten Mikrokügelchen, umfassend spezifische Bindungsstellen, **gekennzeichnet durch** Polymerisieren von funktionellen Monomeren und Vernetzern in einem Reaktionslösungsmittel in Gegenwart von Prägemolekülen als Templaten in einem tensidfreien Fällungspolymerisationsverfahren, wobei diese Prägemoleküle imstande sind, nicht-kovalente oder reversible kovalente Wechselwirkungen mit den funktionellen Monomeren auszubilden.

2. Verfahren nach Anspruch 1, wobei das Gesamtvolumen der polymerisierbaren Monomere und Vernetzer im Bereich von ungefähr 0,01 bis 20 % des Volumens des Reaktionslösungsmittels gehalten wird.

3. Verfahren nach Anspruch 1 oder 2, wobei das Reaktionslösungsmittel wässrig oder nicht-wässrig ist.

4. Verfahren nach Anspruch 1 oder 2, wobei das Reaktionslösungsmittel aus einer einzelnen Lösungsmittelkomponente oder aus mehreren Lösungsmittelkomponenten zusammengesetzt ist.

5. Verfahren nach Anspruch 1, wobei die funktionellen Monomere die gleiche Funktionalität aufweisen.

6. Verfahren nach Anspruch 1, wobei die funktionellen Monomere verschiedene Funktionalität aufweisen.

7. Verfahren nach Anspruch 1 oder 2, wobei die Löslichkeit der Prägemoleküle in dem Reaktionslösungsmittel durch Ändern der Zusammensetzung des Reaktionslösungsmittels eingestellt wird.

8. Verfahren nach Anspruch 1, wobei die Polymerisation durch Wärme, UV-Strahlung, Gamma-Strahlung und/oder chemisch eingeleitet wird.

9. Verfahren nach Anspruch 1, wobei das Polymerisationsverfahren ein Radikalpolymerisationsverfahren, ein lonenpolymerisationsverfahren, ein Koordinationspolymerisationsverfahren oder ein Stufenwachstumspolymerisationsverfahren ist.

10. Verfahren nach Anspruch 1 oder 2, wobei eine gewünschte Größe der Mikrokügelchen durch Kontrollieren der Keimbildung und des Teilchenwachstumsprozesses erzielt wird.

11. Verfahren nach Anspruch 10, wobei die Kontrolle der Keimbildung und des Teilchenwachstumsprozesses erreicht wird durch Einstellen der Zusammensetzung des funktionellen Monomer/Vernetzer/Lösungsmittel-Systems und/oder der Reaktionsbedingungen während der Polymerisation, um die Löslichkeit der wachsenden Polymerketten zu ändern.

12. Verfahren nach Anspruch 10, wobei die Kontrolle der Keimbildung und des Teilchenwachstumsprozesses so ist, dass eine Aggregation der Mikrokügelchen vermieden wird.

13. Verfahren nach Anspruch 1 oder 2, wobei die Größe der hergestellten Mikrokügelchen im Bereich von 0,01-10 µm liegt.

14. Verfahren nach Anspruch 1 oder 2, wobei die Reaktionsbedingungen so kontrolliert werden, dass die Mikrokügelchen monodispers werden.

15. In-vitro-Verwendung der molekular geprägten Mikrokügelchen, hergestellt nach einem der Ansprüche 1-14, zum Durchsuchen von chemischen Bibliotheken, für eine Katalyse, zum Erleichtern einer Synthese, zur Analytbestimmung unter Verwendung von Ligandenbindungsassays und/oder Agglutinationsassays, für therapeutische Zwecke oder für eine kontrollierte Freigabe.

16. Verwendung der molekular geprägten Mikrokügelchen, hergestellt nach einem der Ansprüche 1-14, als stationäre Phase oder Modifikator bei einer Kapillarelektrophorese, Kapillarelektrochromatografie oder HPLC-Analyse.

17. Verwendung der molekular geprägten Mikrokügelchen, hergestellt nach einem der Ansprüche 1-14, als Erkennungskomponente in biomimetischen Sensoren.

18. In-vitro-Verwendung der molekular geprägten Mikrokügelchen, hergestellt nach einem der Ansprüche 1-14, als affinitätsmarkierte Sonde zur gezielten Ansteuerung von Zellen oder anderem biologischen Material.

19. Verwendung der molekular geprägten Mikrokügelchen, hergestellt nach einem der Ansprüche 1-14, als Bindungseinheiten für die Herstellung von Verbundmaterialien.

## Revendications

1. Procédé de production de microsphères à empreinte moléculaire comprenant des sites de liaison spécifiques, **caractérisé par** la polymérisation de monomères fonctionnels et d'agents de réticulation dans un solvant de réaction en présence de molécules à imprimer en tant que modèles dans un processus de polymérisation par précipitation sans agent tensioactif, lesquelles molécules à imprimer sont capables de former des interactions non covalentes ou covalentes réversibles avec lesdits monomères fonctionnels.

2. Procédé selon la revendication 1, dans lequel le volume total des monomères polymérisables et des agents de réticulation est maintenu dans la gamme comprise entre environ 0,01 et 20 % du volume du solvant de réaction.

3. Procédé selon la revendication 1 ou 2, dans lequel le solvant de réaction est aqueux ou non aqueux.

4. Procédé selon la revendication 1 ou 2, dans lequel ledit solvant de réaction est composé d'un seul composant de solvant ou de multiples composants de solvant.

5. Procédé selon la revendication 1, dans lequel lesdits monomères fonctionnels ont la même fonctionnalité.

6. Procédé selon la revendication 1, dans lequel lesdits monomères fonctionnels ont des fonctionnalités différentes.

7. Procédé selon la revendication 1 ou 2, dans lequel la solubilité des molécules à imprimer dans le solvant de réaction est ajustée en changeant la composition du solvant de réaction.

8. Procédé selon la revendication 1, dans lequel la polymérisation est induite par chaleur, rayonnement UV, rayonnement γ et/ou chimiquement.

9. Procédé selon la revendication 1, dans lequel ledit processus de polymérisation est un processus de polymérisation radicalaire, un processus de polymérisation ionique, un processus de polymérisation par coordination ou un processus de polymérisation par étapes.

10. Procédé selon la revendication 1 ou 2, dans lequel une taille souhaitée des microsphères est atteinte en contrôlant la nucléation et le processus de croissance des particules.

11. Procédé selon la revendication 10, dans lequel la contrôle de la nucléation et du processus de croissance des particules est atteint en ajustant la composition du monomère fonctionnel/agent de réticulation/système de solvant et/ou les conditions de réaction au cours de la polymérisation afin de changer la solubilité des chaînes polymères en développement.

12. Procédé selon la revendication 10, dans lequel le contrôle de la nucléation et du processus de croissance des particules est tel qu'il évite l'agrégation des microsphères.

13. Procédé selon la revendication 1 ou 2, dans lequel la taille des microsphères telles que produites est dans la gamme de 0,01 à 10 µm.

14. Procédé selon la revendication 1 ou 2, dans lequel les conditions de réaction sont contrôlées de sorte que les microsphères deviennent monodispersées.

15. Utilisation *in vitro* des microsphères à empreinte moléculaire telles que préparées selon l'une quelconque des revendications 1 à 14, pour le criblage de bibliothèque chimique, pour une catalyse, pour faciliter la synthèse, pour la détermination d'un analyte en utilisant des dosages par liaison de ligands et/ou des dosages d'agglutination, à des fins thérapeutiques, ou pour une libération contrôlée.

16. Utilisation des microsphères à empreinte moléculaire telles que préparées selon l'une quelconque des revendications 1 à 14, en tant que phase stationnaire ou modificateur dans l'électrophorèse capillaire, l'électrochromatographie capillaire ou une analyse par HPLC.

17. Utilisation des microsphères à empreinte moléculaire telles que préparées selon l'une quelconque des revendications 1 à 14, en tant que composant de reconnaissance dans les capteurs biomimétiques.

18. Utilisation *in vitro* des microsphères à empreinte moléculaire telles que préparées selon l'une quelconque des revendications 1 à 14, en tant que sonde marquée par affinité pour le ciblage des cellules ou d'autre matériau biologique.

19. Utilisation des microsphères à empreinte moléculaire telles que préparées selon l'une quelconque des revendications 1 à 14, en tant qu'entités de liaisons pour la préparation de matériaux composites.
